# EUROPÄISCHE PATENTSCHRIFT

(11) **EP 0 793 518 B1**
(45) Veröffentlichungstag und Bekanntmachung des Hinweises auf die Patenterteilung: **05.03.2003**
(21) Anmeldenummer: 96927522.1
(22) Anmeldetag: 23.07.1996
(51) Int. Cl.: A61N 2/06

(54) **MAGNETANORDNUNG ZUR THERAPEUTISCHEN ANWENDUNG**
MAGNET ARRANGEMENT FOR THERAPEUTIC USE
SYSTEME MAGNETIQUE DESTINE A UN USAGE THERAPEUTIQUE

(30) Priorität: 25.09.1995 DE 29515302 U
(43) Veröffentlichungstag der Anmeldung: 10.09.1997
(73) Patentinhaber: Rheinmagnet Horst Baermann GmbH, 53819 Neunkirchen-Seelscheid (DE)
(72) Erfinder: Baermann, Horst, D-51429 Bergisch Gladbach (DE)
(74) Vertreter: Stachow, Ernst-Walther, Prof. Dr.
(86) Internationale Anmeldenummer: DE9601395
(87) Internationale Veröffentlichungsnummer: WO97011749

(56) Entgegenhaltungen:
- WO-A-93/13720
- DE-A- 2 510 173
- DE-U- 9 413 430
- FR-A- 2 611 306
- US-A- 4 549 532

## Beschreibung

Die Erfindung betrifft eine Magnetanordnung zur therapeutischen Anwendung, insbesondere zur Behandlung oberflächennaher Haut- oder Gewebebereiche des menschlichen Körpers, mit einem oder mehreren mechanisch zusammenhängenden magnetischen Formkörpern, die eine wirksame Oberfläche bilden, an der oder in deren Nähe das dort vorhandene magnetische Feld zur Anwendung kommt.

Bekannte derartige Magnetanordnungen weisen zum Beispiel homogene Dauermagnetstreifen, die mit einem oder mehreren Polen aufmagnetisiert sind, oder in bestimmten Figuren diskret angeordnete Dauermagnete gleichen Magnetwerkstoffs auf. Bei den Dauermagneten bzw. Dauermagnetstreifen kann es sich um starre, zum Beispiel aus gesintertem Hartferrit gefertigte Magnete oder auch um flexible Magnete zum Beispiel aus kunststoffgebundenen Ferritpartikeln handeln, wobei die aus handelsüblichem Hartferritpulver bestehenden Partikel in der Kunststoffmatrix, z. B. durch Vermischen in Stempelknetern, in statistisch fein verteilter, homogener Form enthalten sind. Die geometrische Gestalt, Anordnung und Aufmagnetisierung der Formkörper sowie deren Magnetwerkstoff werden entsprechend der beabsichtigten Anwendung ausgewählt. Die an oder in einem bestimmten Abstand von der wirksamen Oberfläche vorhandene magnetische Induktion ist bei bekannten Magnetanordnungen der eingangs genannten Art begrenzt, und es ist in vielen Fällen wünschenswert, die magnetische Induktion an oder in der Nähe der wirksamen Oberfläche zu erhöhen.

In der Therapeutik sind seit längerem Magnetanordnungen mit alternierenden Polen bekannt. Dabei handelt es sich um starre oder flexible Dauermagnete, die auf der dem Körper zugewandten, wirksamen Oberfläche wenigstens einen Pol aufweisen.

Starre, aus gesintertem Hartferrit gefertigte Magnete dieser Art werden vielfach zum Beispiel in Japan verwendet. Daneben haben sich in der Magnettherapeutik seit 1983 in Europa und den USA flexible Magnete aus kunststoffgebundenem Hartferrit durchgesetzt, die auf ihrer dem Körper zugewandten, wirksamen Oberfläche wenigstens einen Nord- und einen Südpol aufweisen, wobei die Polkonfigurationen entsprechend der therapeutischen Zielsetzung unterschiedlich gestaltet sind und von der Firma Rheinmagnet Horst Baermann GmbH unter den Marken "BIOflex" und "VITAflex" vertrieben werden. Derartige Magnetfolien werden in der EP-PS 0 134 437, US-PS 4,549,532 und der JP-PS P1 652 939 beschrieben. Die grundsätzliche Wirksamkeit derartiger Magnetanordnungen auf den menschlichen Organismus konnte in einer von Herrn Dr. Jens Martin von der Klinik Bavaria in Schaufling vorgenommenen Doppelblindstudie nachgewiesen werden.

Bei den für therapeutische Zwecke unter der vorstehend erwähnten Marke "BIOflex" verwendeten Magneten werden maximale Induktionswerte von bis zu ca. 60 mT unmittelbar an der Oberfläche dieser Magnete erreicht. Die zum Erzielen einer therapeutisch relevanten Wirkung erforderliche Induktion von mindestens 1 mT kann dabei jedoch, je nach Materialstärke dieser Magnete, nur bis zu einem Luftspalt (identisch mit der Eindringtiefe in den Körper) von maximal 20 mm erreicht werden. In Abhängigkeit von der therapeutischen Zielsetzung sind nicht nur in oberflächennahen Haut- bzw. Gewebebereichen, sondern auch in tieferen Bereichen von mehr als 7 mm, insbesondere jedoch von mehr als 15 mm von der Körperoberfläche, höhere magnetische Induktionen erwünscht.

Höhere magnetische Induktionen sind aber insbesondere bei großflächigen Anwendungen nicht immer verträglich. Es ist ferner in vielen Fällen wünschenswert, auch magnetisch induzierte Wirkungen in tiefer gelegenen Körperbereichen punktförmig zu erzielen, um solche Wirkungen zu erreichen, wie sie von der Akupunktur her bekannt sind. Da die Induktionswerte im allgemeinen mit der dritten Potenz zum Abstand vom Magneten abnehmen, ist es daher zum Aufbau einer genügend hohen Induktion in tiefer gelegenen Körperbereichen von mehr als 20 mm zwingend erforderlich, Magnete mit besonders hoher Oberflächeninduktion zu verwenden.

Der Erfindung liegt daher die Aufgabe zugrunde, leistungsstärkere Magnetanordnungen der eingangs genannten Art zu schaffen, mit welchen insbesondere hohe lokale Induktionswerte in bestimmten Bereichen der wirksamen Oberfläche sowie eine größere Eindringtiefe in den Körper in diesen Bereichen erzielbar sind.

Die Aufgabe wird erfindungsgemäß dadurch gelöst, daß bei der Magnetanordnung mindestens ein erster magnetischer Formkörper und mindestens ein mit diesem mechanisch zusammenhängender zweiter magnetischer Formkörper vorgesehen sind, wobei die Formkörper eine hohe Koerzitivfeidstärke aufweisen, der Maximalwert des Produkts B · H ([BH]ₘₐₓ-Wert) des ersten Formkörpers geringer als derjenige des zweiten Formkörpers ist und die Formkörper eine bezüglich der beabsichtigten Wirkung des magnetischen Feldes wirksame Oberfläche der Magnetanordnung bilden.

Aufgrund der erfindungsgemäß vorgesehenen, zu einer System- bzw. Wirkungseinheit zusammengefaßten Formkörper unterschiedlicher Energiedichte wird im Bereich der wirksamen Oberfläche des zweiten Formkörpers mit höherer Energiedichte ein stärkerer magnetischer Fluß erreicht, während er im Bereich der wirksamen Oberfläche des ersten magnetischen Formkörpers geringerer Energiedichte schwächer ist. Je nach geometrischer Gestalt, Anordnung und Magnetisierung der Formkörper und Wahl des magnetischen Werkstoffes können somit höhere magnetische Induktionen in bestimmten Bereichen der wirksamen Oberfläche oder im Abstand von dieser erzielt werden.

Mit der Magnetanordnung gemäß der Erfindung können beispielsweise ganz gezielt bestimmte Haut- oder Gewebepartien lokal mit hoher Induktion und damit einhergehender hoher Tiefenwirkung behandelt werden. Beispielsweise lassen sich so gezielt bestimmt Akupunkturpunkte des menschlichen Körpers behandeln. Durch die Erhöhung der Induktion in nur bestimmten Bereichen bleiben im übrigen andere Körperpartien von den Einwirkungen des stärkeren magnetischen Feldes verschont.

Durch die Magnetanordnung gemäß der Erfindung lassen sich vorteilhafterweise therapeutische Zielsetzungen sowohl in oberflächennahen Haut- bzw. Gewebebereichen als auch in örtlich begrenzten Bereichen mit Tiefenwirkung "simultan" realisieren. Dadurch, daß beide unterschiedlichen Magnete eine möglichst hohe Koerzitivfeldstärke aufweisen und mechanisch zusammenhängend ausgebildet sind, kann in vorteilhafter Weise eine gegenseitige Beeinflussung oder Schwächung des einen durch den anderen Magnetwerkstoff weitestgehend vermieden werden.

Die Erfindung schließt die Verwendung mechanisch zusammenhängender Formkörper mit zwei oder mehreren unterschiedlichen [BH]ₘₐₓ-Werten ein.

Vorzugsweise werden die unterschiedlichen Energiedichten der Formkörper durch unterschiedliche Magnetwerkstoffe erreicht. Beispielsweise bestehen der oder die Formkörper mit geringeren Energiedichten aus Barium- oder Strontiumferriten, während die Formkörper mit höheren Energiedichten aus der Gruppe der seltenen Erd-Magnete, wie beispielsweise SmCo₅ oder NdFeB, ausgewählt sind.

Vorzugsweise ist der [BH]ₘₐₓ-Wert des zweiten Formkörpers mindestens zweimal größer als derjenige des ersten Formkörpers. Besonders gute Effekte konnten mit Anordnungen erzielt werden, bei denen der [BH]ₘₐₓ-Wert des zweiten Formkörpers mindestens fünf zehnmal größer ist als derjenige des ersten Formkörpers.

Der zweite Formkörper mit höherer Energiedichte kann in den ersten Formkörper mit niedrigerer Energiedichte an dessen wirksamer Oberfläche eingebettet sein. Auf diese Weise läßt sich eine miteinander fluchtende wirksame Oberfläche der beiden Formkörper erreichen.

Andererseits kann der zweite Formkörper auch auf der wirksamen Oberfläche des ersten Formkörpers als flaches Teil angeordnet sein, so daß die wirksamen Oberflächen beider Formkörper versetzt zueinander angeordnet sind. Da in den meisten Fällen die magnetische Induktion in einem Abstand von der Oberfläche zur Anwendung kommt, läßt sich diese Anordnung meist einfacher herstellen.

Gemäß der Erfindung können in den ersten Formkörper oder auf dessen wirksamer Oberfläche auch mehrere Formkörper höherer Energiedichte, die auch unterschiedlich sein können, angeordnet sein. Ebenfalls kann die Magnetanordnung auch mehrere Formkörper niedrigerer Energiedichte, in bzw. auf denen, wie vorstehend beispielhaft beschrieben, Formkörper höherer Energiedichte befestigt sind, umfassen.

Vorzugsweise ist die wirksame Oberfläche des zweiten Formkörpers bzw. der Formkörper mit höherer Energiedichte kleiner als diejenige des ersten Formkörpers bzw. der Formkörper mit niedrigerer Energiedichte.

Um Streuungen des Magnetfeldes außerhalb des Bereichs über der wirksamen Oberfläche möglichst zu vermeiden, ist zweckmäßigerweise die Vektorsumme aller magnetischen Flüsse auf der wirksamen Oberfläche der Magnetanordnung annähernd null.

Der zweite Formkörper kann zum Beispiel in eine Aussparung oder Bohrung des ersten Formkörpers formschlüssig eingesetzt und durch bekannte Befestigungsmöglichkeiten, wie Verkleben, Verschweißen oder beidseitiges Abdecken mit einer Klebeschicht oder dergleichen, fixiert werden. Wegen der magnetischen Streufelder ist jedoch ein formschlüssiges Einpassen nicht unbedingt erforderlich. So kann es zur Schaffung größerer Raumstreuung des magnetischen Feldes sogar vorteilhaft sein, zwischen den beiden Formkörpern einen Luftspalt geeigneter Abmessungen vorzusehen.

Bei getrennter Anordnung der Formkörper ist es zweckmäßig, diese auf der der wirksamen Oberfläche gegenüberliegenden Seite durch eine ferromagnetische Rückschlußschicht, zum Beispiel aus kohlenstoffarmem Weicheisen oder eine starre oder flexible Schicht aus nichtmagnetisierbarem Material, wie zum Beispiel unmagnetischem Edelstahl, Aluminium oder einem Kunststoff oder aus einer Kombination dieser Bestandteile, zu verbinden.

Andererseits können die räumlich getrennt voneinander angeordneten Formkörper auch an deren wirksamer Oberfläche durch eine starre oder flexible Schicht aus nichtmagnetisierbarem Material, zum Beispiel mit den vorstehend erwähnten Bestandteilen, miteinander verbunden sein. Dabei kommen starre oder elastische bzw. flexible Schichten in Betracht. Über die Verbindung der Formkörper hinaus dienen sie der Formstabilität der Magnetanordnung.

Zur Befestigung des zweiten Formkörpers auf der wirksamen Oberfläche des ersten kann zweckmäßigerweise ebenfalls eine aus nichtmagnetisierbarem Material bestehende Zwischenschicht, beispielsweise aus einem Kleber, einer Kunststoffschicht oder einem beliebigen anderen nichtmagnetisierbaren Material, wie etwa einem Gewebe, vorgesehen sein.

Gemäß der Erfindung, die auf eine größtmögliche Streuwirkung des magnetischen Feldes im Bereich über der wirksamen Oberfläche ausgerichtet ist, werden solche Magnetanordnungen bevorzugt, bei denen der erste Formkörper bzw. die Formkörper mit geringerer Energiedichte in axialer Richtung, d. h. senkrecht zu ihrer wirksamen Oberfläche, magnetisiert sind. Dabei kann der Formkörper je nach beabsichtigter Anwendung an seiner wirksamen Oberfläche einen oder mehrere Pole, gegebenenfalls mit abwechselnder Polarität und in beliebiger Anordnung, aufweisen. Die Formkörper müssen nicht notwendigerweise in axialer Richtung magnetisiert sein, die Erfindung ist so zu verstehen, daß jedwede Art der Magnetisierung in Betracht kommt.

Gemäß einer besonders bevorzugten Ausführung der Erfindung werden solche Magnetanordnungen vorgeschlagen, bei denen der erste Formkörper an seiner wirksamen Oberfläche mit einem Pol aufmagnetisiert ist oder aus mit Abstand zueinander angeordneten Teilkörpern mit aufmagnetisierten Polen gleichen Vorzeichens besteht und der zweite Formkörper vorzugsweise mehrere in den ersten Formkörper eingebettete bzw. zwischen dessen Polen angeordnete oder auf dessen Oberfläche befestigte Teilkörper gleicher, jedoch von dem ersten Formkörper verschiedener Polarität an deren wirksamen Oberflächen aufweist, die in an sich bekannten geometrischen Figuren und Anordnungen ausgebildet sind.

Im allgemeinen kann der erste Formkörper an seiner wirksamen Oberfläche auch mit mehreren Polen aufmagnetisiert sein oder aus mit Abstand zueinander angeordneten Teilkörpern mit aufmagnetisierten Polen unterschiedlicher Polarität bestehen, wobei der zweite Formkörper, wie vorstehend beschrieben, zwischen zwei Polen des ersten Formkörpers angeordnet ist und die an den beiden Formkörpern gegenüberliegenden Pole unterschiedliche Polarität aufweisen. Die Magnetanordnung besteht auch in diesem Fall aus einer Polkonfiguration mit Polen abwechselnder Polarität, wobei in den Bereichen des oder der Formkörper höherer Energiedichte an der wirksamen Oberfläche höhere magnetische Induktionen erhalten werden und wobei im übrigen die Polflächen der Formkörper die an sich bekannten geometrischen Gebilde und Anordnungen, wie zum Beispiel streifenförmige, zirkulare, radiale, drei- oder mehreckige, segment-, sichel- bzw. sonnenradförmige und ähnliche Ausbildungen aufweisen.

Einige Ausführungsbeispiele der Erfindung werden nachstehend anhand der Zeichnung näher erläutert. In der Zeichnung zeigen:
- Fig. 1: ein Ausführungsbeispiel der Magnetanordnung für therapeutische Zwecke,
- Fig. 2: einen Schnitt längs der Linie II-II in Fig. 1,
- Fig. 3: einen Schnitt durch ein zweites Ausführungsbeispiel,
- Fig. 4: ein weiteres Ausführungsbeispiel der Magnetanordnung,
- Fig. 5: eine Abwandlung des Ausführungsbeispiels gemäß Fig. 4,,
- Fig. 6: ein anderes Ausführungsbeispiel der Magnetanordnung,
- Fig. 7: ein weiteres Ausführungsbeispiel der Magnetanordnung,
- Fig. 8: eine Draufsicht auf die Magnetanordnung gemäß Fig. 7,
- Fig. 9: eine Abwandlung des Ausführungsbeispiels gemäß Fig. 7,
- Fig. 10 - Fig. 14: weitere Ausführungsbeispiele für Magnetanordnungen mit unterschiedlichen Pol-Konfigurationen der betreffenden Formkörper, wobei die Fig. 13 eine Schnittansicht entlang der Linien XIII-XIII in Fig. 12 zeigt.

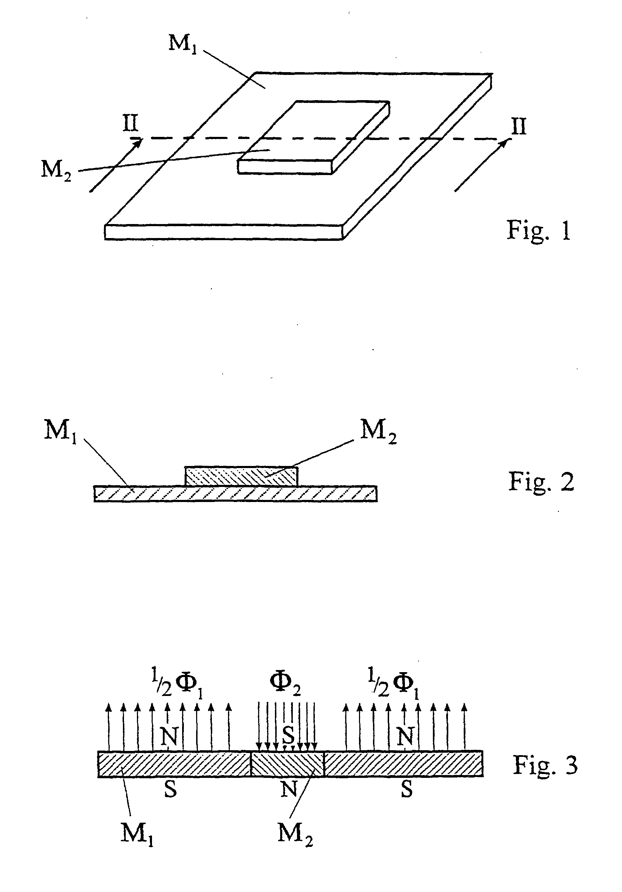

Die in den Fig. 1 bis 3 gezeigten Magnetanordnungen bestehen aus einem ersten magnetischen Formkörper M₁ und einem mit diesem mechanisch zusammenhängenden zweiten magnetischen Formkörper M₂, wobei die beiden Formkörper M₁ und M₂ eine hohe Koerzitivfeldstärke aufweisen, der [BH]ₘₐₓ-Wert des ersten Formkörpers M₁ geringer als derjenige des zweiten Formkörpers M₂ ist und die beiden Formkörper M₁ und M₂ eine bezüglich der beabsichtigten Wirkung des magnetischen Feldes wirksame Oberfläche (Oberseite der in den Fig. 1 bis 3 dargestellten Magnetanordnungen) bilden. Bei der in den Fig. 1 und 2 gezeigten Ausführung ist der zweite Formkörper M₂ auf der wirksamen Oberfläche des ersten Formkörpers M₁ als flaches Teil angeordnet.

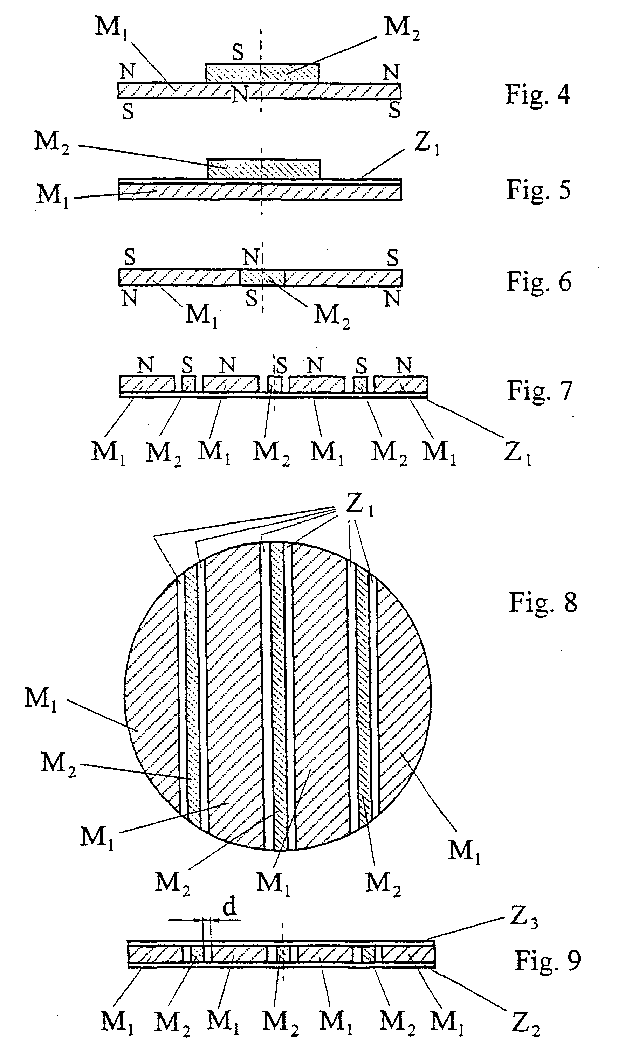

Bei der in Fig. 3 dargestellten Ausführung ist der zweite Formkörper M₂ in den ersten Formkörper M₁ an dessen wirksamer Oberfläche eingebettet.

Bei beiden Ausführungen ist die wirksame Oberfläche des zweiten Formkörpers M₂ kleiner als diejenige des ersten Formkörpers M₁. Wie weiterhin aus Fig. 3 hervorgeht, ist die Vektorsumme aller magnetischen Flüsse auf der wirksamen Oberfläche der beiden Formkörper M₁ und M₂ null.

Die beiden folgenden Beispiele zeigen unterschiedliche Magnetwerkstoffe für die Formkörper M₁ und M₂ und die dazugehörigen Werte für die magnetische Remanenz Bᵣ, die Koerzitiv-Feldstärke ᵢH_{c} und das maximale magnetische Energieprodukt [BH]ₘₐₓ.

### Beispiel 1:

M₂ besteht aus isotropem kunststoffgebundenem NdFeB.

M₁ besteht aus kunststoffgebundenem isotropem Ba-Ferrit

| | M₂ | M₁ |
|---|---|---|
| Bᵣ [mT] | 470 | 163 |
| ᵢH_{c} [kA/m] | 700 | 240 |
| [BH]ₘₐₓ [kJ/m³] | 35 | 4,5 |

### Beispiel 2:

M₂ besteht aus isotropem gesintertem NdFeB.

M₁ besteht aus anisotropem kunststoffgebundenem Sr-Ferrit.

| | M₂ | M₁ |
|---|---|---|
| Bᵣ [mT] | 1.250 | 220 |
| ᵢH_{c} [kA/m] | 800 | 280 |
| [BH]ₘₐₓ [kJ/m³] | 280 | 9,0 |

Als besonders vorteilhaft zur Erzielung einer therapeutischen Wirkung haben sich die in den Figuren 4 bis 14 dargestellten Ausführungsformen erwiesen, die nachstehende beispielhaft beschrieben werden. Dabei ist in allen Fällen der magnetisch höherwertige Formkörper M₂, der aus einem Dauermagnetwerkstoff mit Elementen der Gruppe der seltenen Erden, wie beispielsweise NdFeB oder SmCo₅, besteht, flächen- und volumenmäßig kleiner als der magnetisch geringerwertige Formkörper M₁, der beispielsweise aus Sr-Ferrit besteht. In den Ausführungsbeispielen werden kreisrunde bzw. scheibenförmige Magnete verwendet. Es können jedoch auch ebenso beliebig andere geometrische Figuren, wie Ovale, Rechtecke, Fünf-, Sechs- und Mehrecke, oder sonstige symmetrische oder auch asymmetrische flächige Gebilde verwendet werden.

Bei den in den Fig. 4 und 5 gezeigten Magnetanordnungen ist wenigstens ein Formkörper M₂ höherer Energiedichte auf der Oberfläche eines Formkörpers M₁ niedrigerer Energiedichte durch direkten Kontakt (Fig. 4) oder aber auch in Sonderfällen durch eine Zwischenschicht Z₁, beispielsweise einem Kleber, einer Kunststoffschicht oder einem beliebigen anderen nichtmagnetisierbaren Medium, wie etwa einem Gewebe, angeordnet (Fig. 5).

Die Magnetanordnung nach Fig. 6, die grundsätzlich schon anhand Fig. 3 beschrieben wurde, weist wenigstens einen Formkörper M₂ größerer Energiedichte auf, der in einem Formkörper M₁ niedriger Energiedichte integriert ist. Der Formkörper M₂ ist in eine Bohrung im Formkörper M₁ eingelassen und dort durch geeignete Befestigungsmöglichkeiten, wie Verkleben, Verschweißen oder beidseitiges Abdecken mit Klebeplättchen und dergleichen, fixiert.

Um größere Raumstreuungen des magnetischen Feldes zu erhalten, ist gemäß der in den Fig. 7 und 8 gezeigten Magnetanordnung ein Abstand zwischen den Formkörper M₁ und M₂ vorgesehen. In den erwähnten Figuren sind mehrere streifenförmige Formkörper M₂ höherer Energiedichte zwischen mehreren streifenförmigen Formkörpern M₁ geringerer Energiedichte angeordnet. Die wirksame Oberfläche der Formkörper M₁ ist größer als diejenige der Formkörper M₂.

Der jeweilige Abstand zwischen den Formkörpern M₁ und M₂ beträgt vorzugsweise bis zu 50 mm. Die aus unterschiedlichen Magnetwerkstoffen bestehenden Formkörper M₁ und M₂ werden durch eine starre oder elastische bzw. flexible Verbindungsschicht Z₁ miteinander zu einer kreisrunden Magnetanordnung verbunden.

Bei der in Fig. 9 gezeigten räumlich voneinander getrennten Anordnung der Formkörper M₁ und M₂ ist an der der wirksamen Oberfläche gegenüberliegenden Seite eine ferromagnetische Rückschlußschicht Z₂ aus zum Beispiel kohlenstoffarmem Weicheisen vorgesehen. Weiterhin ist an der wirksamen Oberfläche der Formkörper M₁ und M₂ eine starre oder flexible Schicht Z₃ aus nichtmagnetisierbarem Material, wie unmagnetischem Edelstahl, Aluminium oder einem Kunststoff oder aus einer Kombination dieser Bestandteile, angeordnet, die im wesentlichen der Formstabilität der Magnetanordnung dient.

Für die vorstehend beschriebenen prinzipiellen Magnetanordnungen ist es unerheblich, welche Polkonfiguration die Formkörper M₁ und M₂ an ihrer wirksamen Oberfläche haben. Bei den dargestellten Ausführungen ist der Formkörper M₁ in axialer Richtung, d. h. senkrecht zu seiner wirksamen Oberfläche, magnetisiert, um eine größtmögliche Streuwirkung des magnetischen Feldes zu erhalten.

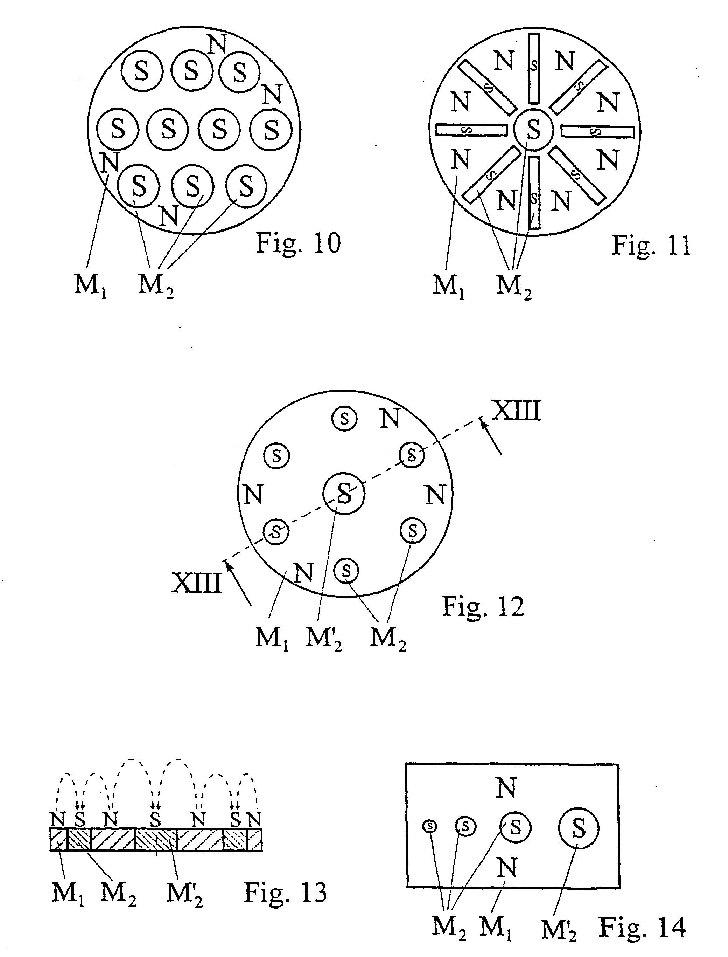

Die Ausführungsbeispiele gemäß Fig. 10 bis Fig. 14 betreffen andere kreisrunde bzw. rechteckige Magnetanordnungen, bei denen magnetische Formkörper M₂ höherer Energiedichte mit einem oder mehreren Formkörpern M₁ geringerer Energiedichte nach den vorstehend beschriebenen und in den Fig. 4 bis 9 gezeigten Anordnungen eine mechanisch zusammenhängende Wirkungseinheit bilden. Die Fig. 12 bis 14 zeigen zwei Ausführungen, die mehrere Formkörper, beispielsweise M₂ und M₂', größerer Energiedichte aufweisen, wobei sich diese Formkörper sowohl in ihren geometrischen Abmessungen und Konfigurationen als auch in ihrer Energiedichte unterscheiden können. Auf diese Weise läßt sich die magnetische Flußdichte an der wirksamen Oberfläche und das magnetische Streufeld in bestimmten Bereichen verstärken und variieren, so daß diese Größen dem jeweiligen Anwendungsfall zur Erzielung bestimmter Wirkungen noch besser angepaßt werden können.

## Patentansprüche

1. Magnetanordnung zur therapeutischen Anwendung, insbesondere zur Behandlung oberflächennaher Haut- oder Gewebebereiche des menschlichen Körpers, mit mindestens einem ersten magnetischen Formkörper (M₁) und mindestens einem mit diesem mechanisch zusammenhängenden zweiten Formkörper (M₂), wobei die Formkörper (M₁,M₂) eine hohe Koerzitiv-Feldstärke aufweisen und eine bezüglich der beabsichtigten Wirkung des magnetischen Feldes wirksame Oberfläche der Magnetanordnung bilden, **dadurch gekennzeichnet, daß** der Maximalwert des Produktes B · H, [BH]ₘₐₓ-Wert, des ersten Formkörpers (M₁) geringer als derjenige des zweiten Formkörpers (M₂) ist.

2. Magnetanordnung nacht Anspruch 1, **dadurch gekennzeichnet, daß** die beiden Formkörper (M₁, M₂) aus unterschiedlichen Magnetwerkstoffen bestehen.

3. Magnetanordnung nach Anspruch 2, **dadurch gekennzeichnet, daß** der erste Formkörper (M₁) aus Barium- oder Strontiumferriten und der zweite Formkörper (M₂) aus der Gruppe der seltenen Erden besteht.

4. Magnetanordnung nach einem der Ansprüche 1 bis 3, **dadurch gekennzeichnet, daß** der [BH]ₘₐₓ-Wert des zweiten Formkörpers (M₂) mindestens zweimal größer ist als derjenige des ersten Formkörpers (M₁).

5. Magnetanordnung nach Anspruch 4, **dadurch gekennzeichnet, daß** der [BH]ₘₐₓ-Wert des zweiten Formkörpers (M₂) mindestens fünfzehmnal größer ist als derjenige des ersten Formkörpers (M₁).

6. Magnetanordnung nach einem der Ansprüche 1 bis 5, **dadurch gekennzeichnet, daß** der zweite Formkörper (M₂) in den ersten Formkörper (M₁) an dessen wirksamer Oberfläche eingebettet ist.

7. Magnetanordnung nach einem der Ansprüche 1 bis 5, **dadurch gekennzeichnet, daß** der zweite Formkörper (M₂) auf der wirksamen Oberfläche des ersten Formkörpers (M₁) als flaches Teil angeordnet ist.

8. Magnetanordnung nach Anspruch 6 oder 7, **dadurch gekennzeichnet, daß** die wirksame Oberfläche des zweiten Formkörpers (M₂) kleiner als diejenige des ersten Formkörpers (M₁) ist.

9. Magnetanordnung nach einem der Ansprüche 1 bis 8, **dadurch gekennzeichnet, daß** die Vektorsumme aller magnetischen Flüsse auf der wirksamen Oberfläche der gesamten Magnetanordnung annähernd null ist.

10. Magnetanordnung nach Anspruch 6, **dadurch gekennzeichnet, daß** der zweite Formkörper (M₂) formschlüssig in den ersten Formkörper (M₁) eingesetzt ist.

11. Magnetanordnung nach Anspruch 6, **dadurch gekennzeichnet, daß** zwischen dem ersten und zweiten Formkörper (M₁ bzw. M₂) ein Luftspalt angeordnet ist.

12. Magnetanordnung nach Anspruch 11, **dadurch gekennzeichnet, daß** die beiden Formkörper (M₁, M₂) auf der der wirksamen Oberfläche gegenüberliegenden Seite durch eine ferromagnetische Rückschlußschicht (Z₂) oder eine starre oder flexible Schicht (Z₁) aus nichtmagnetisierbarem Material miteinander verbunden sind.

13. Magnetanordnung nach Anspruch 11 oder 12, **dadurch gekennzeichnet, daß** die beiden Formkörper (M₁, M₂) an der wirksamen Oberfläche durch eine starre oder flexible Schicht (Z3) aus nichtmagnetisierbarem Material miteinander verbunden sind.

14. Magnetanordnung nach Anspruch 7, **dadurch gekennzeichnet, daß** der zweite Formkörper (M₂) durch eine aus nichtmagnetisierbarem Material bestehende Zwischenschicht (Z₁) mit dem ersten Formkörper (M₁) verbunden ist.

15. Magnetanordnung nach einem der Ansprüche 6 bis 14, **dadurch gekennzeichnet, daß** der erste Formkörper (M₁) in axialer Richtung, d. h. senkrecht zu seiner wirksamen Oberfläche, magnetisiert ist.

16. Magnetanordnung nach einem der Ansprüche 1 bis 15, **dadurch gekennzeichnet, daß** der erste Formkörper (M₁) ein starrer Dauermagnet aus gesintertem Hartferrit ist.

17. Magnetanordnung nach einem der Ansprüche 1 bis 15, **dadurch gekennzeichnet, daß** der erste Formkörper (M₁) ein flexibler Dauermagnet aus kunststoffgebundenem Hartferrit ist.

18. Magnetanordnung zur therapeutischen Anwendung nach einem der Ansprüche 1 bis 17, **dadurch gekennzeichnet, daß** der erste Formkörper (M₁) an seiner wirksamen Oberfläche mit einem Pol aufmagnetisiert ist oder aus mit Abstand zueinander angeordneten Teilkörpern mit aufmagnetisierten Polen gleicher Polarität besteht und der zweite Formkörper (M₂) mehrere in den ersten Formkörper (M₁) eingebettete bzw. zwischen dessen Polen angeordnete oder auf dessen Oberfläche befestigte Teilkörper gleicher, jedoch von dem ersten Formkörper (M₁) verschiedener Polarität an deren wirksamen Oberflächen aufweist, die in an sich bekannten geometrischen Figuren und Anordnungen ausgebildet sind.

## Claims

1. Magnetic arrangement for therapeutic application, particularly for the treatment of surface near skin or tissue areas of the human body, with at least one first magnetic body (M₁) and at least one second magnetic body (M₂) being mechanically connected to the first, whereby the magnetic bodies (M₁, M₂) show a high coercive field strength and, reference the intended effect of the magnetic field, form an active surface of the magnetic arrangement, wherein the maximum value of the products B H, [BH]ₘₐₓ-value, of the first body (M₁) is lower than that of the second body (M₂).

2. Magnetic arrangement as per Claim 1, wherein both magnetic bodies (M₁, M₂) consist of different magnetic material.

3. Magnetic arrangement as per Claim 2, wherein the first magnetic body (M₁) consists of barium or strontium ferrites and the second magnetic body (M₂) consists of a group of the rare earths.

4. Magnetic arrangement as per Claims 1 to 3, wherein the [BH]ₘₐₓ-value of the second magnetic body (M₂) is at least double that of the first magnetic body (M₁).

5. Magnetic arrangement as per Claim 4, wherein the (BH]ₘₐₓ-value of the second body (M₂) is at least fifteen times that of the first magnetic body (M₁).

6. Magnetic arrangement as per Claims 1 to 5, wherein the second magnetic body (M₂) is embedded into the active surface of the first magnetic body (M₁).

7. Magnetic arrangement as per one of Claims 1 to 5, wherein the second magnetic body (M₂) is arranged on the active surface of the first magnetic body (M₁) in the form of a flat piece.

8. Magnetic arrangement as per Claims 6 or 7, wherein the active surface of the second magnetic body (M₂) is smaller than that of the first magnetic body (M₁).

9. Magnetic arrangement as per one of Claims 1 to 8, wherein the vector sum of all magnetic fluxes on the active surface of the total magnetic arrangement is nearly zero.

10. Magnetic arrangement as per Claim 6, wherein the second magnetic body (M₂) is form-fit embedded into the first magnetic body (M₁).

11. Magnetic arrangement as per Claim 6, wherein an airgap is arranged between the first and the second magnetic body (M₁ respectively M₂).

12. Magnetic arrangement as per Claim 11, wherein both magnetic bodies (M₁, M₂) are connected to one another on the side opposite to the active surfaces, by a ferro-magnetic retainer layer (Z₂) or a rigid, or flexible, layer (Z₁) of non-magnetizable material.

13. Magnetic arrangement as per Claim 11 or 12, wherein both magnetic bodies (M₁, M₂) are connected to one another on the active surface by a rigid, or flexible, layer (Z₃) of non-magnetizable material.

14. Magnetic arrangement as per Claim 7, wherein the second magnetic body (M₂) is connected to the first magnetic body (M₁) with an intermediate layer (Z₁) of non-magnetizable material.

15. Magnetic arrangement as per one of Claims 6 to 14, wherein the first magnetic body (M₁) is magnetized in axial direction, i.e. vertical to its active surface.

16. Magnetic arrangement as per one of Claims 1 to 15, wherein the first magnetic body (M₁) is a rigid permanent magnet of sintered hard ferrite.

17. Magnetic arrangement as per one of Claims 1 to 15, wherein the first magnetic body (M₁) is a flexible permanent magnet of plastic bonded hard ferrite.

18. Magnetic arrangement for therapeutic application as per one of Claims 1 to 17, wherein the first magnetic body (M₁) is magnetized with one pole on its active surface, or consists of body segments with magnetized poles of identical polarity, arranged apart from one another, and the second magnetic body (M₂) has several magnetic body segments of identical polarity, but differing to those on the active surface of the first magnetic body (M₁), which are arranged in known geometrical figures and arrangements embedded in the first magnetic body (M₁), or arranged between its poles, or fixed to its surface.

## Revendications

1. Agencement magnétique pour application thérapeutique, en particulier pour traitement de zones épidermiques ou tissulaires proches de la surface du corps humain, comportant au moins un premier corps conformé magnétique (M₁) et au moins un deuxième corps conformé (M₂) groupé à celui-ci par voie mécanique, les corps conformés (M₁, M₂) présentant une haute intensité de champ coercitif et formant une surface de l'agencement magnétique, laquelle est efficace par rapport à l'effet intentionnel du champ magnétique, **caractérisé en ce que** la valeur maximale du produit B H, c'est-à-dire la valeur [BH]ₘₐₓ, du premier corps conformé (M₁) est inférieure à celle du deuxième corps conformé (M₂).

2. Agencement magnétique selon la revendication 1, **caractérisé en ce que** les deux corps conformés (M₁, M₂) sont constitués de matériaux magnétiques différents.

3. Agencement magnétique selon la revendication 2, **caractérisé en ce que** le premier corps conformé (M₁) est constitué de ferrites de baryum ou de strontium et le deuxième corps conformé (M₂) est constitué d'un élément parmi le groupe des terres rares.

4. Agencement magnétique selon l'une des revendications 1 à 3, **caractérisé en ce que** la valeur [BH]ₘₐₓ du deuxième corps conformé (M₂) est au moins deux fois plus grande que celle du premier corps conformé (M₁).

5. Agencement magnétique selon la revendication 4, **caractérisé en ce que** la valeur [BH]ₘₐₓ du deuxième corps conformé (M₂) est au moins quinze fois plus grande que celle du premier corps conformé (M₁).

6. Agencement magnétique selon l'une des revendications 1 à 5, **caractérisé en ce que** le deuxième corps conformé (M₂) est noyé dans le premier corps conformé (M₁) à sa surface efficace.

7. Agencement magnétique selon l'une des revendications 1 à 5, **caractérisé en ce que** le deuxième corps conformé (M₂) est agencé comme pièce plate sur la surface efficace du premier corps conformé (M₁).

8. Agencement magnétique selon l'une ou l'autre des revendications 6 et 7, **caractérisé en ce que** la surface efficace du deuxième corps conformé (M₂) est inférieure à celle du premier corps conformé (M₁).

9. Agencement magnétique selon l'une des revendications 1 à 8, **caractérisé en ce que** la somme vectorielle de tous les flux magnétiques sur la surface efficace de l'ensemble de l'agencement magnétique est approximativement nulle.

10. Agencement magnétique selon la revendication 6, **caractérisé en ce que** le deuxième corps conformé (M₂) est intégré en coopération de formes dans le premier corps conformé (M₁).

11. Agencement magnétique selon la revendication 6, **caractérisé en ce qu'**un entrefer est prévu entre le premier et le deuxième corps conformé (M₁ ou M₂).

12. Agencement magnétique selon la revendication 11, **caractérisé en ce que** les deux corps conformés (M₁, M₂) sont reliés l'un à l'autre sur le côté opposé à la surface efficace par une couche de retour ferromagnétique (Z₂) ou par une couche rigide ou flexible (Z₁) en matériau non magnétisable.

13. Agencement magnétique selon l'une ou l'autre des revendications 11 et 12, **caractérisé en ce que** les deux corps conformés (M₁, M₂) sont reliés l'un à l'autre sur la surface efficace par une couche rigide ou flexible (Z₃) en matériau non magnétisable.

14. Agencement magnétique selon la revendication 7, **caractérisé en ce que** le deuxième corps conformé (M₂) est relié au premier corps conformé (M₁) par une couche intermédiaire (Z₁) constituée en matériau non magnétisable.

15. Agencement magnétique selon l'une des revendications 6 à 14, **caractérisé en ce que** le premier corps conformé (M₁) est magnétisé en direction axiale, c'est-à-dire perpendiculairement à sa surface efficace.

16. Agencement magnétique selon l'une des revendications 1 à 15, **caractérisé en ce que** le premier corps conformé (M₁) est un aimant permanent rigide en ferrite dure frittée.

17. Agencement magnétique selon l'une des revendications 1 à 15, **caractérisé en ce que** le premier corps conformé (M₁) est un aimant permanent flexible en ferrite dure liée par de la matière plastique.

18. Agencement magnétique pour application thérapeutique selon l'une des revendications 1 à 17, **caractérisé en ce que** le premier corps conformé (M₁) est magnétisé avec un pôle sur sa surface efficace ou est constitué par des corps partiels agencés à distance les uns des autres et présentant des pôles magnétisés de même polarité, et **en ce que** le deuxième corps conformé (M₂) comprend plusieurs corps partiels noyés dans le premier corps conformé (M₁) ou agencés entre ses pôles ou fixés sur sa surface, qui présentent sur leurs surfaces efficaces la même polarité qui diffère cependant de celle du premier corps conformé (M₁), surfaces qui sont réalisées sous forme de figures et de dispositions géométriques connues en soi.
